(19) Europäisches Patentamt
European Patent Office
Office européen des brevets

(11) Publication number: **0 415 748 A1**

# (12) EUROPEAN PATENT APPLICATION

(21) Application number: **90309475.3**

(22) Date of filing: **30.08.90**

(51) Int. Cl.[5]: **C07D 319/06**, C07D 407/12, C07D 249/08, C07C 69/78, C07C 33/46, C07C 43/178, C07C 309/66

(30) Priority: **01.09.89 US 402166**

(43) Date of publication of application: **06.03.91 Bulletin 91/10**

(84) Designated Contracting States: **GR**

(71) Applicant: **SCHERING CORPORATION**
**2000 Galloping Hill Road**
**Kenilworth New Jersey 07033(US)**

(72) Inventor: **Girijavallabhan, Viyyoor M.**
**10 Maplewood Drive**
**Parsippany, New Jersey 07054(US)**
Inventor: **Ganguly, Ashit K.**
**96 Cooper Avenue**
**Upper Montclair, New Jersey 07043(US)**
Inventor: **Versace, Richard W.**
**65 B Townsend Road**
**Wanaque, New Jersey 07465(US)**

(74) Representative: **Ritter, Stephen David et al**
**Mathys & Squire 10 Fleet Street**
**London EC4Y 1AY(GB)**

## (54) Threonine derived asymmetric chemical synthesis and intermediates for making antifungal compounds.

(57) Intermediates of the structural formula III or Va having an asymmetric center as indicated by an asterisk (*) and being substantially free of its opposite enantiomeric form:

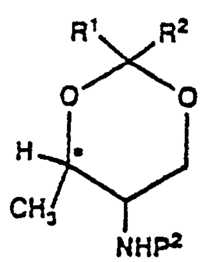

III or

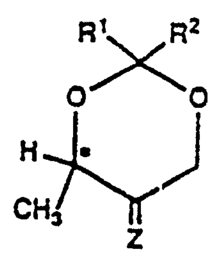

Va

wherein:
Z represents O or $NOP^1$
$P^1$ is

EP 0 415 748 A1

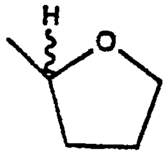 , 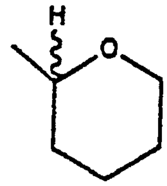 , 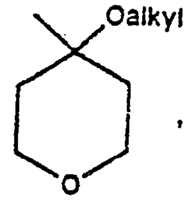 ,

H, alkyl, benzyl, substituted benzyl, alkoxy-substitued phenyl, alkyloxyalkyl, alkylthioalkyl or R'R"R'"silyl.

$R^1$ and $R^2$ may be the same or different and each independently represents H, alkyl, phenyl, substituted phenyl or $R^1$ and $R^2$ together with the carbon to which they are attached represent the group

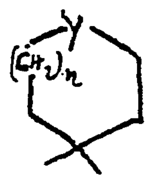

Y is O, $S(O)_p$ or $(CR^aR^b)$ ,

Y is O, $S(O)_p$ or $(CR^aR^b)$, and processes for making chemically pure, enantiomeric antifungal compounds using a threonine derived asymmetric chemical synthesis.

# THREONINE DERIVED ASYMMETRIC CHEMICAL SYNTHESIS AND INTERMEDIATES FOR MAKING ANTIFUNGAL COMPOUNDS

European Published Application Nos. 178533 and 61835 disclose certain antifungal compounds having two asymmetric centers of the formula:

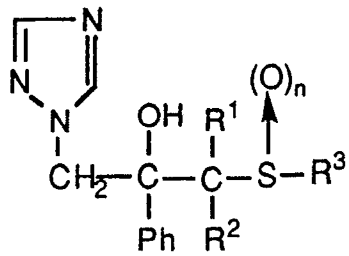

wherein Ph is a phenyl group or a phenyl group substituted with one or two halogen atoms, $R^1$ is a $C_1$-$C_3$ alkyl group, $R^2$ is a hydrogen atom or a $C_1$-$C_3$ alkyl group, $R^3$ is a $C_1$-$C_8$ alkyl group, a $C_4$-$C_8$ cycloalkylalkyl group or a $C_3$-$C_6$ cycloalkyl group and n is 0, 1 or 2, and their acid addition salts. Certain of these isomers have been found to provide more advantageous properties than the racemic mixture from which they are derived. Neither published application, however, discloses any asymmetric chemical syntheses for making the individual isomers. It would be highly advantageous to provide processes and intermediates by which the individual, pure isomers could be prepared economically and in good yield.

U.S. Patent No. 4,526,983 discloses a process for making certain optical active imidazolyl propanoate antifungal compounds having the formula

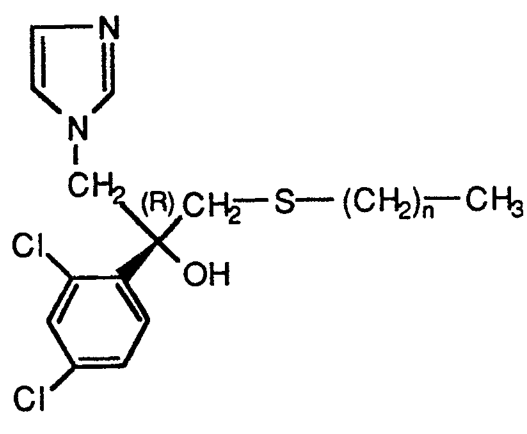

wherein n is an integer of 3 or 4, and its acid addition salts, by diastereomeric separation using an optically active carboxylic acid ester. This separation process is not suitable for large scale commercial synthesis. It is laborious, low yielding and expensive.

The present invention involves a series of important, novel compounds useful as intermediates in making chemically pure, enantiomeric compounds having antifungal activity. One aspect of the invention involves a novel intermediate compound of the formula III or Va having an asymmetric center as indicated by the asterisk (*) (i.e., an R or S absolute stereochemical configuration) and being substantially free of its opposite enantiomeric form:

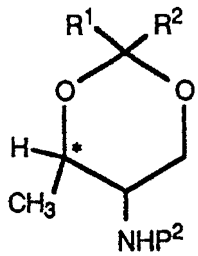 III or 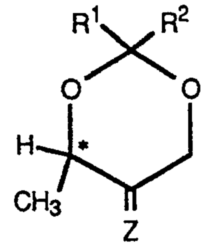 Va

wherein:
Z represents O or $NOP^1$;
$P^1$ is

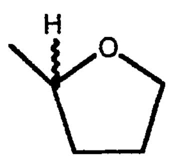, 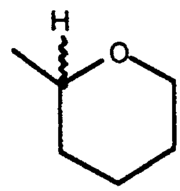, 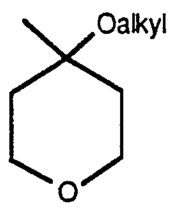,

H, alkyl, benzyl, substituted benzyl, alkoxy-substituted phenyl, alkyloxyalkyl, alkylthioalkyl or $R'R''R'''$silyl where $R'$, $R''$ and $R'''$ may be the same or different and each is selected from alkyl, cycloalkyl or phenyl;
$R^1$ and $R^2$ may be the same or different and each independently represents H, alkyl, phenyl, substituted phenyl or $R^1$ and $R^2$ together with the carbon to which they are attached represent the group

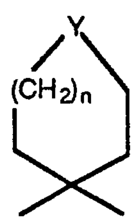 ;

Y is O, $S(O)_p$ or $(CR^aR^b)$;
$R^a$ and $R^b$ may be the same or different and each is independently selected from H, alkyl, alkoxy, phenyl, substituted phenyl, or -COOH or an alkyl, phenyl or substituted phenyl ester thereof,
p is 0, 1 or 2;
n is 0, 1 or 2; and
$P^2$ represents H or an amino protecting group.

Another intermediate compound of the invention has the structural formula VII, wherein both asterisks (*) represent the same absolute stereochemical configuation (i.e., RR or SS forms), and wherein the compound is substantially free from compounds having other absolute stereochemical configurations at the asterisked (*) carbon centers (i.e., if the asterisks in formula VII indicate an RR form, the compound is substantially free from its SS, SR and RS forms):

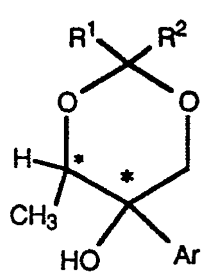 VII

wherein Ar represents phenyl, substituted phenyl, 2- or 3-thienyl, substituted 2- or 3-thienyl, 2- or 3-furanyl, substituted 2- or 3-furanyl, 2-, 4-or 5-imidazolyl, 3-or 5-(1,2,4-triazolyl), 5-tetrazolyl, 2-, 4-or 5-thiazolyl, 2-, 4- or 5-oxazolyl, 3-, 4-or 5-isoxazolyl, 2-,3-or 4-pyridinyl, 2-or 3-pyrrolyl, 3-or 4-pyrrazolyl, 2-benzimidazolyl, 2-benzthiazolyl, or 2-, 4-or 6-purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl; and wherein as in $R^1$ and as in $R^2$ are as defined above.

Compounds III, Va and VII also have other possibly asymmetric carbons, i.e., the carbon attached to $R^1$ and $R^2$ or the carbon attached to $NHP^2$ in formula III. The absolute stereochemical configuration at these other carbon centers is not limited by the present invention since in the processes described below the specific stereoconfiguration at these other carbon atoms does not affect the desired end product.

A preferred novel intermediate compound of the invention is of the formula Va, wherein Z is O or NOH. $R^1$ is preferably H and $R^2$ is preferably phenyl or substituted phenyl. Preferably, the compound of formula Va at the asterisk (*) carbon atom is in its R absolute stereochemical configuration.

Another preferred intermediate of the invention is of formula VII. $R^1$ is preferably H and $R^2$ is preferably phenyl. Ar is preferably substituted phenyl, more preferably Ar is mono, di or tri- substituted halo phenyl, e.g., 2,4-difluoro, 2,6-difluoro, 4-fluoro, 2-fluoro, 2,4-dichloro, 2,6-dichloro, 4-chloro or 2-chloro substituted phenyl. A particularly preferred Ar group is 2,4-difluorophenyl. Both asymmetric centers indicated by the asterisk (*) are preferably in their R absolute stereochemical configuration.

The above novel intermediate compounds provide very advantageous starting matreials for making the desired antifungal end products having the formula XVI

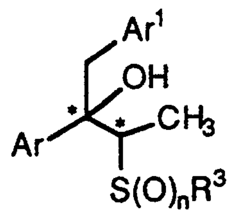

XVI

wherein both of the asymmetric carbon centers indicated by the asterisks (*) have the same absolute stereochemical configuration, and wherein said compound is substantially free from compounds having other absolute stereochemical configurations at such carbon centers;

wherein Ar represents phenyl, substituted phenyl, 2- or 3-thienyl, substituted 2- or 3-thienyl, 2- or 3-furanyl, substituted 2- or 3-furanyl, 2- ,4-or 5-imidazoyl, 3-or 5-(1,2,4-triazolyl), 5-tetrazolyl, 2-,4- or 5-thiazolyl, 2-, 4- or 5-oxazolyl, 3-,4- or 5-isoxazolyl, 2-benzthiazolyl, or 2-,4-or 6-purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl;

wherein $Ar^1$ represents 1-(1,2,4-triazolyl), 1-(1,3,4-triazolyl) or 1-imidazolyl;

$R^3$ is alkyl, cycloalkyl or cycloalkylalkyl; and

wherein n is 1 or 2.

In the processes described below, Ar, $R^1$, $R^2$, $P^1$ and $P^2$ are as defined above unless otherwise indicated. Wavy lines indicate mixtures of the possible isomers and the asterisks (*) indicate (here and throughout this specification) an asymmetric carbon center in its absolute stereochemical R or S form, which is substantially free of its opposite enantiomeric form. The double asterisk (**) represents an asymmetric carbon center which has the opposite absolute stereochemical configuration than an asterisk (*) carbon in the scheme below. For example, it the single asterisk (*) indicates an absolute stereochemical R form, the double asterisks (**) indicates an absolute stereochemical S form and vice versa.

Thus, one process aspect of the invention involves reacting a compound of the formula I having an asymmetric carbon center as indicated by the asterisk (*) and being substantially free of its opposite enantiomeric form:

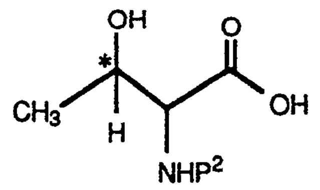

I

or an ester thereof with a reducing agent to form a compound of the formula II

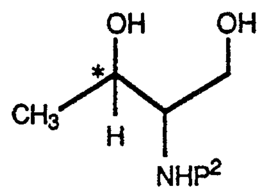

II;

and reacting the compound of formula II with an acetal or ketal forming agent of the formula $R^1R^2CO$, $R^1R^2C(Oalkyl)_2$ or $R^2CHO$ to form a compound of the formula III

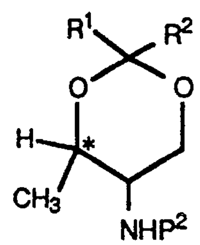

III.

The compound of formula I is generally commercially available or easily obtainable and inexpensive. The absolute stereochemical configuration sof the asymmetric carbons attached to $NHP^2$ and to $R^1$ and $R^2$ are not important in the process of the invention. The former group is converted to a Z group as explained below and the latter group is eliminated prior to obtaining the desired end product.

The compound of formula III can then be reacted with a deprotecting agent (when $P^2$ is not H) to form a compound of formula IV

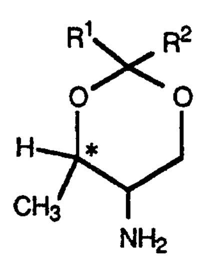

IV;

which is then reacted with an oxidizing agent, followed if desired by reaction with a hydroxy protecting agent when $P^1$ is not H, to form a compound of the formula V

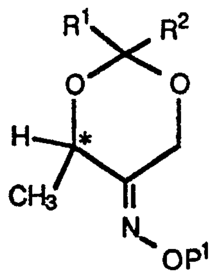

V.

The compound of formula V can then be further oxidized to form a compound of formula VI

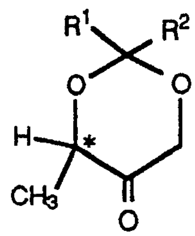

VI.

This cyclic ketone structure of formula VI surprisingly maintains its enantiomeric integrity at the the asterisked (*) carbon. In open chain compounds, by contrast, racemization of an alpha-hydroxy ketone occurs rapidly when such ketones are treated with basic reagents.

The compound of formula VI may be reacted with a compound of the formula ArM where M represents a metallic group to form a compound of formula VII and a lesser amount of a compound of formula VIIa

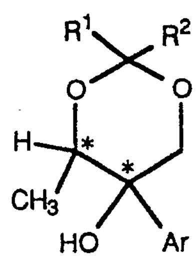

VII or

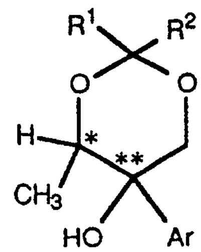

VIIa,

where both of the asterisks in formula VII have the same absolute stereochemical configuration (i.e., RR or SS form), while the single asterisk (*) carbon and the double asterisk (**) carbon in formula VIIa have the opposite stereochemical configuration, i.e., the single asterisk represents an R form and the double asterisk (**) an S form or single asterisk (*) represents an S form and double asterisk (**) an R form. Surprisingly, the compound of formula VII is obtained as the major product contrary to the literature (J. Am. Chem. Soc., 1987, Vol. 109, (pp. 908-910) which indicates that the opposite enantiomeric form would be obtained. Thus, if one starts with compound I with the asterisk (*) in an R absolute stereochemical configuration and follows the above series of reaction steps, then one would get compound VII as the major component with both the asterisk asymmetric centers having R absolute stereochemical configuration and a minor component VIIa having the single asterisk (*) and double asterisk (**) carbon atoms with R and S absolute stereochemical configuration, respectively. In VII and VIIa R stereochemical configuration is maintained at the carbon bearing the methyl group and the new asymmetric center created by the addition of ArM to the carbonyl function in formula VI possesses either R or S stereochemical configuration, thus resulting in formula VII or VIIa, respectively.

The compound of formula VII may also be prepared by reacting the compound of formula V with an $Ar^1$ anion to form a compound of the formula VIII where the asterisk asymmetric centers (*) are of the same absolute stereochemical configuration:

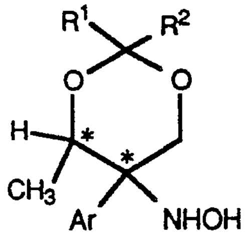

VIII;

which is then reacted with a nitrosating agent such as $NaNO_2$ in mild acid to form the compound of the formula VII.

The compound of formula VII can be reacted (when R1 represents H) with an oxidizing agent, such as N-halo-succinimide or halogen-water, to form a compound of formula IX where $Z^1$ is O and X is halogen, with a reducing agent (when RI represents H) to form a compound of formula IX where $Z^1$ is $H_2$ and X is OH, or with a hydrolyzing agent to form a triol of the formula IXa:

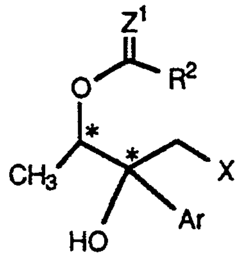

IX or

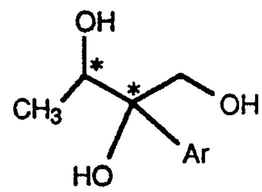

IXa.

The primary hydroxy group in formula IX (if X is OH) or in IXa is first converted to a leaving group such as mesylate or halo group, and then the compound of the formula IX or IXa is reacted with an $Ar^1$ anion to form a compound of the formula XII or XIII, respectively:

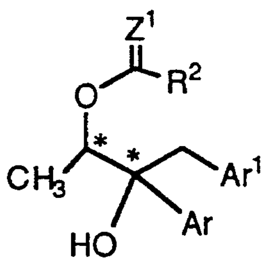

XII or

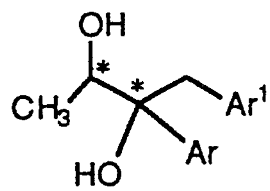

XIII,

wherein $Ar^1$ represents 1-(1,2,4-triazolyl), 1-(1,3,4-triazolyl) or 1-imidazolyl and wherein $Z^1$ represents H,H or O.

The compound of formula VIIa may be reacted with a mesylating agent to form a compound of the formula X wherein Ms represents a mesyl group ($CH_3SO_2$-) and the carbon centers having the single asterisk (*) and the double asterisks (**) are of opposite absolute stereochemical configurations

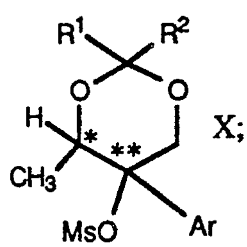

X;

Compound of formula X in turn is reacted (when $R^1$ is H) with an oxidizing agent such as a peracid, ozone, selenium oxide,etc., followed by base treatment, to form a compound of the formula XI where $Z^1$ is O, with a reducing agent (when $R^1$ is H) followed by base treatment to form a compound of formula XI where $Z^1$ is

$H_2$, or with a hydrolyzing agent followed by base treatment to form a compound of formula XIa:

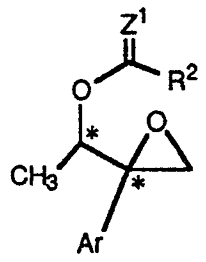 XI or 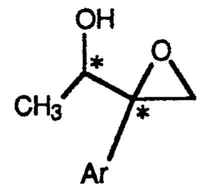 XIa.

The stereochemistry in the compounds of formulas XI and XIa are inverted at the carbon attached to the Ar group with respect to the same carbon atom in the compounds of formula X. The compounds of the formula XI and XIa are reacted with an $Ar^1$ anion to form a compound of the formula XII or XIII, respectively.

The compound of formula XIII may then be employed to prepare the desired antifungal end products of formula XVI by applying the methods known per se such as those described in European published application No. 0178533 by reacting the compound of formula XIII with a mesylating agent to form a compound of the formula XIV

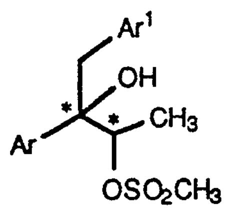 XIV;

reacting the compound of the formula XIV with mercaptan anion of the formula $R^3S^-$ to form a compound of the formula XV

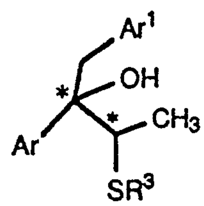 XV;

where $R^3$ is alkyl, cycloalkyl or cycloalkylalkyl; and reacting the compound of the formula XV with an oxidizing agent to form a compound of the formula XVI

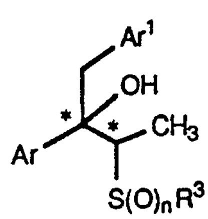 XVI,

wherein n is 1 or 2. $Ar^1$ is preferably 1-(1,2,4-triazolyl), Ar is preferably 2,4-difluorophenyl, $R^3$ is preferably methyl, n is preferably 2, and the asterisks preferably indicate the RR absolute stereochemical form of the compound. Using similar chemistry and starting with a compound of formula I having carbon the asterisk (*) in an S absolute stereochemical configuration, compound XVI could be obtained possessing S absolute stereochemical configuration at both asterisked (*) asymmetric carbons (i.e. SS).

This invention relates to substantially pure enantiomers and to processes for making such substantially pure enantiomers. By substantially pure, we mean substantially free from any of the other enantiomeric forms of such compounds, e.g., one enantiomer is present in amounts greater than about 95 molar %, more

preferably greater than about 97 molar %, most preferably greater than about 99 molar %, with respect to any other enantiomer.

The following terms when used herein have the meanings given below, unless otherwise indicated:

acyl - represents alkanoyl, halo-substituted alkyl carbonyl, benzoyl and substituted benzoyl;

alkyl (including the alkyl portions of alkanoyl, alkylthio, alkoxy, alkyloxyalkyl, alklythioalkyl, alkanoyloxy, etc.) - represents a straight or branched hydrocarbon chain having from 1 to 12, preferably from 1 to 6, carbon atoms;

amino protecting group - represents a group which will protect an amino group during a desired chemical reaction such as the reactions described above, e.g., suitable amino protecting groups include benzyl, substituted benzyl, alkoxy-substituted phenyl, acyl, alkoxycarbonyl, halo-substituted alkoxycarbonyl, unsubstituted or substituted allyloxycarbonyl, etc.;

cycloalkyl (including the cycloalkyl portion of cycloalkylalkyl) - represents a saturated carbocyclic ring having from 3 to 8, preferably from 3 to 6, carbon atoms;

substituted cycloalkyl - represents a cycloalkyl group as defined above wherein one or two of the hydrogens on different carbons of the ring are replaced with a substitutent selected from halo, alkyl, alkoxy, alkylthio, phenoxy, alkanoyl, benzoyl, alkanoyloxy, benzoyloxy, dialkylamino, etc.;

halo - represents fluoro, chloro, bromo or iodo;

hydroxy protecting group - represents a group which will protect the hydroxy group during a desired chemical reaction such as the reactions described above, e.g., suitable hydroxy protecting groups include benzyl, substituted benzyl, alkoxy-substituted phenyl, acyl, alkoxycarbonyl, halo-substituted alkoxycarbonyl, or unsubstituted or substituted allyloxycarbonyl,

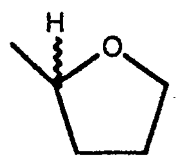 , 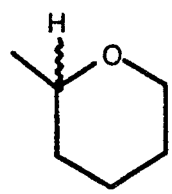 , 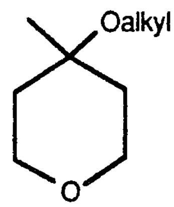 ,

alkyl, alkyloxyalkyl, alkylthioalkyl or $R'R''R'''$silyl where $R'$, $R''$ and $R'''$ may be the same or different and each is selected from alkyl, cycloalkyl or phenyl;

substituted phenyl, substituted benzyl and substituted benzoyl - represent groups in which the phenyl rings thereof are substituted with one or more subsituent groups each independently selected from halo, alkyl, hydroxyalkyl, hydroxy, alkoxy, alkylthio, phenoxy, alkanoyl, benzoyl, alkanoyloxy, benzoyloxy, amino, alkylamino, dialkylamino, etc.; and

substituted allyloxycarbonyl - represents an allyloxycarbonyl group in which the allyl group is substituted with halo, phenyl, substituted phenyl, etc.

The processes and novel intermediate compounds of the invention are illustrated in the following schematic reaction sequence, wherein Ar, $Ar^1$, $R^1$, $R^2$, $R^3$, $P^1$, $P^2$, $Z^1$ and n are as defined above and the single asterisk (*) and the double asterisk (**) have the meanings described above:

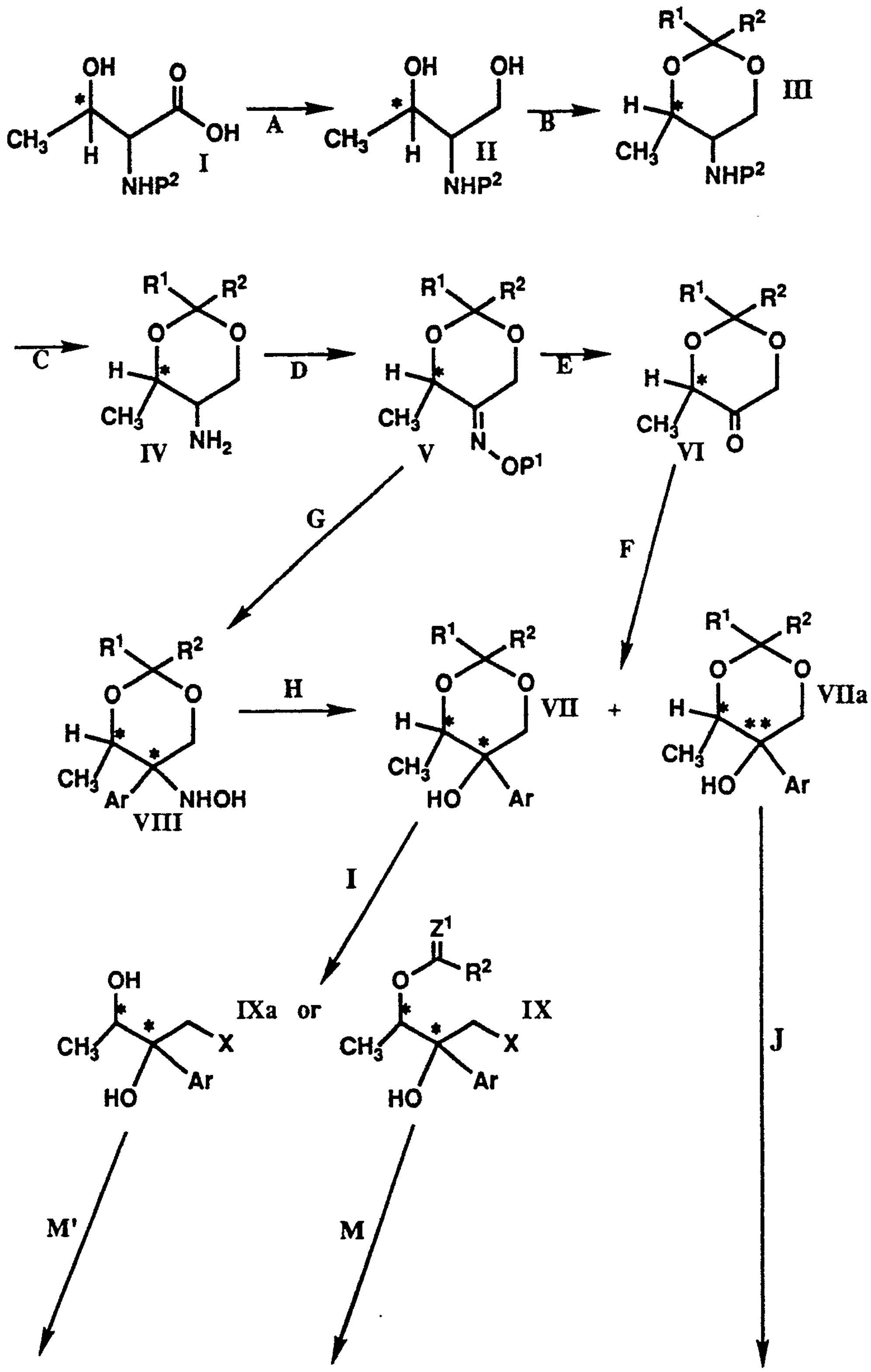


I II III IV V VI VIII VII + VIIa IXa or IX

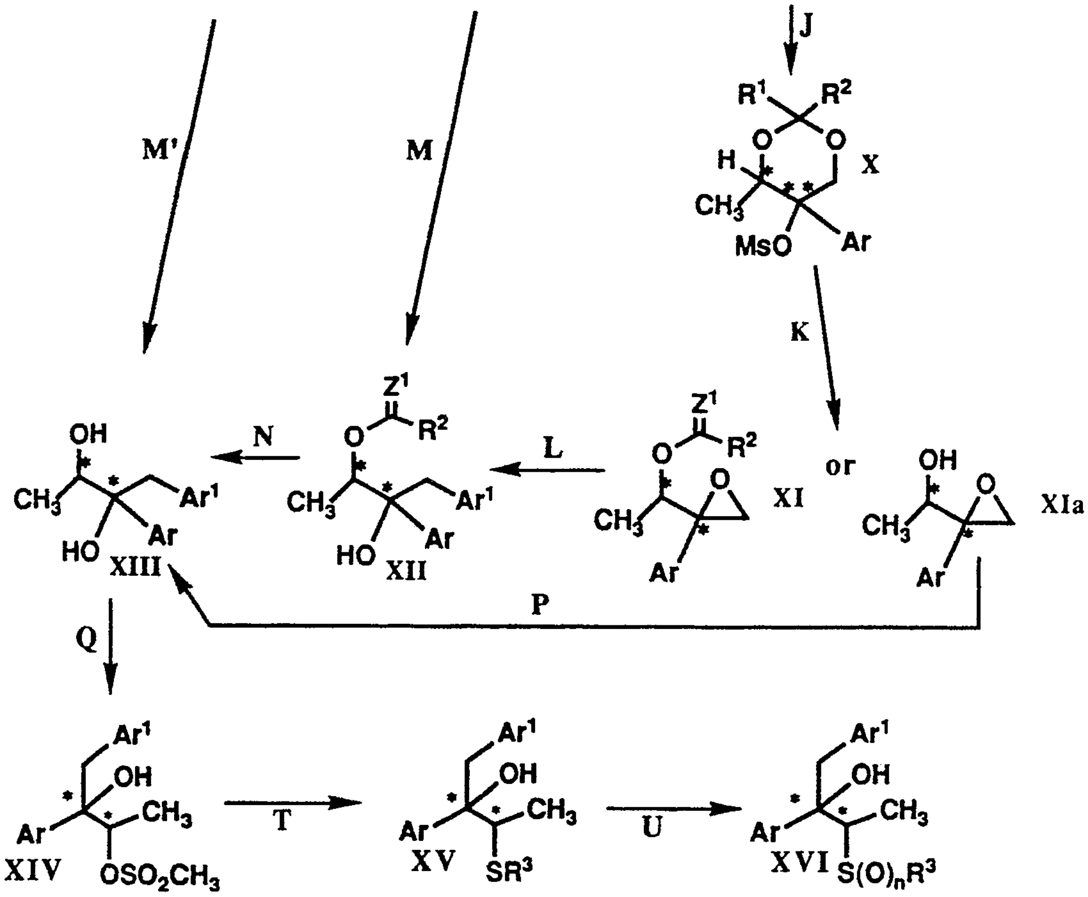


The asymmetric carbon center indicated by single asterisk (*) in formula I is preferably in its R absolute stereochemical form and will thus result in an end product of formula XVI having R absolute stereochemical configuration at both of the asterisked (*) carbon centers, i.e., the RR form of the compound of formula XVI. If the compound of formula I has the S absolute stereochemical configuration at the asterisked (*) carbon, both of the carbon atoms indicated by the asterisk (*) in the final product XVI will be in their S absolute stereochemical configuration, i.e., the SS form of the compound of formula XVI.

In Step A, the carboxylic acid group or its suitably protected form (e.g., an ester) is reduced to a hydroxyl group. Any suitable reducing agent can be employed, e.g., lithium aluminium hydride (when formula I is in ester form) or diborane (when formula I is in the acid form) in a non-participating solvent such as tetrahydrofuran, diethylether and the like. The temperature of the reaction may range from about 0° C to a refluxing temperature, preferably about room temperature. When the reduction is complete, excess reagent may be neutralized, e.g, using dilute acetic acid or aqueous $NH_4Cl$ solution. In the above operation the free amino group is temporarily protected either as a benzoyl group or as a benzyl group for the convenience of the extractive work up. This protecting group is usually removed before Step B by a hydrogenative process.

In Step B, the amino group of formula II is usually protected by any suitable protecting group, e.g., an acyl group, preferably by a trifluoroacetyl group. Then both of the hydroxyl groups are protected as an acetal or ketal group by reaction with an acetal or ketal forming agent of the formula $R^1R^2CO$, $R^1R^2C$-(Oalkyl)$_2$ or $R^2CHO$. The reaction is usually conducted under acid catalysis at temperatures ranging from about 0° C to about 100° C in any suitable solvent such as $CH_2Cl_2$, toluene, etc. The reaction is worked up by neutralizing to about pH 7 and separating the organic layer in a conventional manner.

In Step C, the amine protecting group in formula III is removed. If it is an acyl group, it is removed by conventional base hydrolysis (e.g., $NaOH/H_2O$), in a suitable solvent such as ethanol, acetonitrile, etc., at about 0° C to about 60° C, preferably at about room temperature. The product may be extracted into an organic solvent such as methylene chloride, diethyl ether, etc.

In Step D, the amino group of formula IV is oxidized to an oxime group (in formula V) or directly to a carbonyl group (as in formula VI). The oxidation can be performed using $H_2O_2$ in presence of sodium tungstate ($Na_2WO_4$) or by hypochlorites or by quinones. The oxidation is usually performed in aqueous medium in presence of a miscible organic solvent such as ethanol, methanol, dimethylsulfoxide (DMSO),

dimethylformamide (DMF), etc. The temperature of the reaction can be from about 0°C to about 80°C, preferably at about room temperature. The product usually crystallizes out of the medium. The amount of the water miscible solvent is to be adjusted to retain most of the desired product out of solution.

In Step E, the oxime of formula V (which may be protected by a suitable protecting group $P^1$) is further oxidized using a nitrosating agent such as sodium nitrite. The reaction is usually performed in an aqueous medium employing an organic miscible solvent such as ethanol, DMF, DMSO, acetonitrile, etc., at about 0°C to about 40°C, preferably at about 20 to about 30°C. A mild acid catalyst such as acetic acid usually accelerates the oxidation to the ketone. When the reaction is over, it is neutralized, e.g., to pH 7, and the desired product is extracted into a suitable solvent, e.g., $CH_2Cl_2$. This ketone of formula VI may also be obtained directly from the amine by oxidizing with a quinone reagent and the resultant imine intermediate is then hydrolyzed to the ketone during acid work up.

In Step F, the keto group of formula VI is reacted with an organometallic reagent ArM to obtain the desired adduct. The organometallic reagent can be a Grignard reagent; a lithium derivative of an alkyl or an aryl compound; a zinc, titanium, copper or tin derivative of an alkyl, aryl or substituted alkyl or aryl compound; or a silylated compound from which silicon is removed by fluoride ion to generate the reactive species. The reaction is usually performed in a non-participating solvent such as the aprotic solvents typically used in reactions using organometallic regents, e.g. tetrahydrofuran (THF), diethyl ether, toluene, etc. The temperature of the reaction can be from about -60°C to about +80°C and preferably at about 0°C to about 25°C. The stereochemistry of the addition of ArM to the ketone provides the chirality as RR or SS as the predominant species VII depending on whether the starting ketone of formula VI is in the R or S form, respectively. Minor amounts of RS or SR isomers VIIa formed in the above reaction, depending on whether the starting ketone of formula VI is in the R or S form, respectively, may be utilized by following Steps J and K as further explained below. However, by adjusting the nature of the solvent, e.g., THF, diethyl ether, t-butylmethyl ether, dimethoxyethane, dioxane, etc., and by mixing with metal ions such as $Li^+$, $Mg^{2+}$, $Zn^{2+}$, $Ti^{4+}$, $Zr^{4+}$, $Cu^+$, $K^+$, $Na^+$, $Cs^+$, $Al^{3+}$, etc., one can obtain the desired RR or SS isomer in maximum yield. Crown ethers also may be employed if the organometallic reagent is weakly reactive.

The compound of formula VII may also be obtained from the oxime of formula V as in Step G above by first reacting with ArM under conditions described in Step F. The product of formula VIII formed is then reacted in step H with a nitrosating agent such as $NaNO_2$ in mild acid medium to nitrosate the hydroxyl amine. Loss of $N_2$ gives substantially the desired product of formula VII.

In Step 1, the protecting group of the alcoholic groups (ketal or acetal group) in formula VII can be removed or partially removed as desired to obtain the primary hydroxyl group free for further reaction. For example, reduction in the presence of trimethylsilyl chloride or trimethyl silyl triflate by sodium cyanoborohydride in acetonitrile at about 0°C to about 50°C produces the $R^2CH_2$-ether IX, i.e., where $Z^1 = H_2$ and X = OH. Oxidation with N-bromo succinimide in presence of a radical initiator such as benzoyl peroxide gives the ester IX ($Z^1$ = O and X is Br). Acid hydrolysis in aqueous medium gives the triol IXa. When X = OH, it may be converted into a leaving group such as a mesylate or a halogen (Cl, Br, I) by conventional methods. This group is then further reacted with a nucleophile, displacing the X leaving group in the next step.

The RS or SR diastereomer of formula VIIa is converted to formula X by making the tertiary hydroxy group a leaving group such as mesylate. This is done by conventional reagents such as mesyl chloride in THF in the presence of a base (triethylamine) at about 0°C to about 50°C, preferably at about room temperature.

Step K, which is same as Step I above, plus a base treatment (with for example triethylamine) after Step I, gives the epoxide of formula XI or XIa. Note that stereochemistry at the Ar carbon center in formula XI is inverted during the base catalysed epoxide formation. This epoxide can be directly reacted with a nucleophile as explained below.

The compound of formula XII or XIII is obtained from the compound of formula IX, IXa, XI or XIa in steps M, M′, L and P by reaction with a nucleophile $Ar^1$ anion in a suitable solvent such as DMF, DMSO, $CH_3CN$, etc., at temperatures ranging from about 0°C to about 80°C, preferably at room temperature to about 50°C.

In step N, the protecting group on the oxygen atom in formula XII is removed by conventional methods. For example, when $Z^1$ is O, base hydrolysis ($NaOH/H_2O$)in aqueous ethanol gives the compound of the formula XIII. When $Z^1$ is H,H, catalytic hydrogenation provides the compound of the formula XIII. The compound of the formula XIII may also be obtained by reacting either the compound of formula XIa or IXa with an $Ar^1$ anion under the same conditions as in step M above.

Steps Q, T and U may be performed as described in European published application No. 0178533. For example, the compound of formula XIII is reacted with a mesylating agent such as methanesulfonyl chloride

($CH_3SO_2Cl$) in an inert solvent (e.g., benzene, DMF, dichloromethane, etc.) in the presence of a base at about 0° C. to about 30° C to give the methane sulfonate or mesylate of the formula XIV. The mesylating agent is typically employed in excess (1 to 2 equivalents). The base can be, for example, an organic amine, e.g., pyridine.

The methane sulfonate or mesylate of formula XIV is then reacted in step T with a mercaptan anion of the formula $R^2S^-$ where $R^2$ is alkyl, cycloalkyl or cycloalkylalkyl. The counterion is preferably an alkali metal salt and the solvent is preferably inert (e.g., DMSO, DMF, etc.). The thiol salt may be employed in excess (2 to 10 equivalents) and the temperature is typically 0 to 80° C.

The sulfide of the formula XV is then oxidized in step U using an oxidizing agent such as a peracid, e.g., meta-chloroperbenzoic acid, in an inert solvent, e.g., chloroform, at a temperature of from about -30C to reflux.

EXAMPLE 1

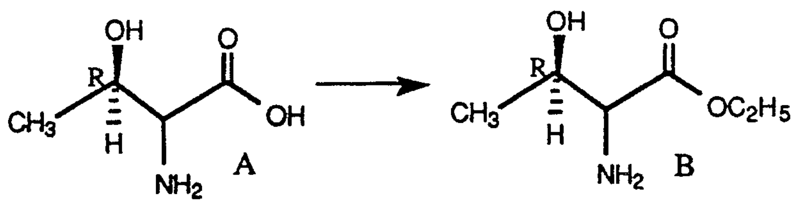

Add L-threonine (A) (100 g) to ethyl alcohol (1000 ml). Add thionyl chloride (125 ml) dropwise over 1 hr. Reflux the mixture for about 3 hrs. Remove the ethyl alcohol by high vacuum. Add 500 ml of ethyl alcohol and 74 g of a 50% by weight sodium hydroxide solution to give a pH of about 9. Remove the solvent by high vacuum. Extract twice with hot ether. fitter off the solid sodium chloride and crystallize the product of formula B from ether. Yield 101.7 g (82%).

EXAMPLE 2

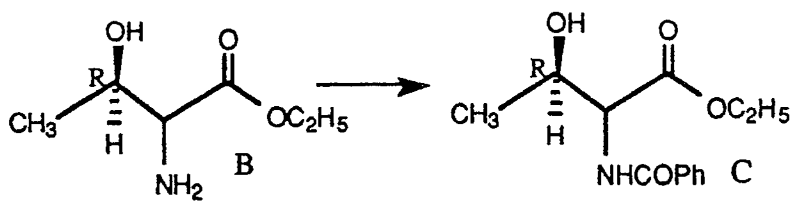

Add sodium carbonate (28 g) and the threonine ester of formula B (25 g) from Example 1 above to a mixture of 250 ml of $H_2O$ and 250 ml of tetrahydrofuran (THF). Cool the reaction mixture to 0° C and add 26 g of benzoyl chloride dropwise over about 1 hr. Add sodium chloride to saturate. Partition the reaction mixture with ethyl acetate. Remove the solvents by vacuum distillation. Recrystallize the solid product of formula C obtained in methylene chloride/hexane. Yield: 42 g.

EXAMPLE 3

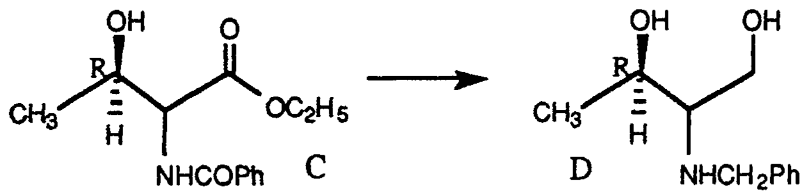

C D

Add lithium aluminum hydride (65 g) to 1000 ml of THF under $N_2$ purge in a 3 liter 3-neck flask. Dissolve the threonine ester of formula C (203 g) from Example 2 above in 500 ml of THF. Cool the lithium aluminum hydride solution in an ice bath and add the threonine ester solution via a double tipped needle using $N_2$ to pressurize the flask. Add the threonine ester solution at a rate that just causes refluxing of the reaction mixture. After complete addition, reflux the mixture for 1 1/2 hours. Cool and stir overnight. Add 50 ml of ethyl acetate to the reaction mixture and then 70 ml of 15% aqueous sodium hydroxide solution. Add 150 ml of $H_2O$. Filter the reaction mixture through a celite plug. Pass the filtrate through a silica gel plug. Pass 10% $CH_3OH/CH_2Cl_2$ to elute the desired compound. Rotovap the eluate to give the desired product of formula D. Yield: 159 g.

## EXAMPLE 4

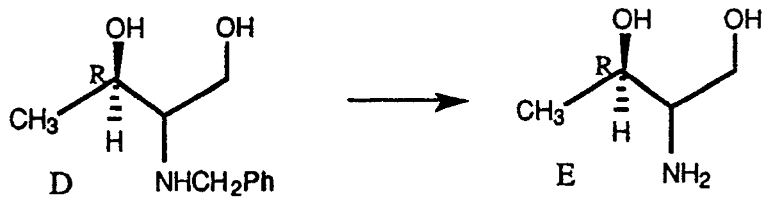

D E

Add the product of formula D from Example 3 above (159 g), 20 g of 10% palladium on carbon and 1 liter of ethyl alcohol to a 2 liter Parr bottle. Pressurize the bottle to 50 psi with $H_2$ until the uptake of $H_2$ stops. Filter off the Pd/C and evaporate the solvent to give the desired product of formula E as a crystalline solid. Yield: 69.5 g.

## EXAMPLE 5

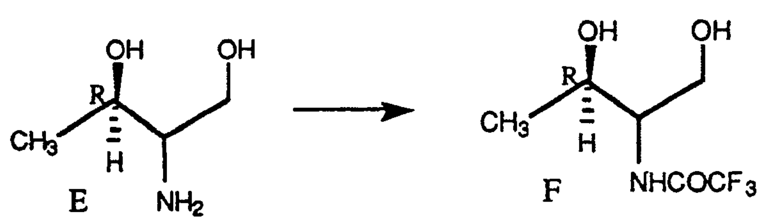

E F

Add methyl alcohol (350 ml) to the aminodiol (69 g) of formula E from Example 4 above. Add ethyl trifluroacetate (135 ml). After about 1 hr., remove the volatile solvents using a rotovap and high vacuum to give the desired product of formula F as a crystalline solid. Yield: 128g.

## EXAMPLE 6

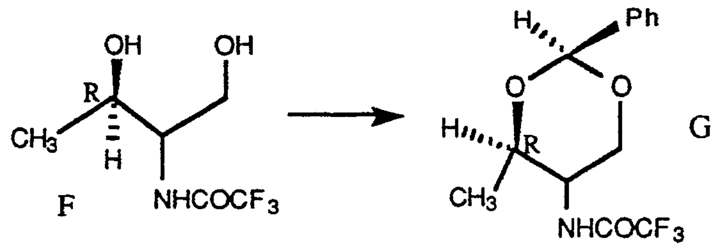

F → G

Add the amidodiol (10 g) of formula F from Example 5 above, benzaldehyde dimethoxyacetal (40 ml) and 0.5 g of pyridine paratoluene sulfonic acid (PPTSA) to 150 ml of toluene. Slowly distill off the methanol and toluene under vacuum (240 mm Hg @ 80 ° C). After about 30 ml of the solvent is collected, the remaining toluene solution is washed with water and then distilled to dryness under high vacuum. The residue is purified on a silica column using a gradient eluant of 25% $CH_2Cl_2$/hexane → 100% $CH_2Cl$ to provide 11.3 g of the desired product of formula G as an oil.

EXAMPLE 7

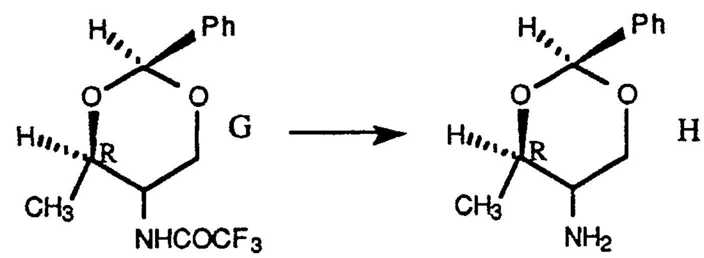

G → H

Add 11 g of the product of formula G from Example 6 above and 125 ml of saturated $NH_3/CH_3OH$ solution to 50 ml of water and stir until all starting material is deprotected as indicated by TLC (silica gel/methylene chloride). Remove the volatile solvents by high vacuum. Extract the reaction mixture with methylene chloride and dry with $Na_2SO_4$. Purify the product of formula H by eluting it from a silica column using a gradient eluant of 100% methylene chloride → 20% $CH_3OH/CH_2Cl_2$), employing $CH_3OH$ saturated with $NH_3$. Yield: 7.2 g (98%).

EXAMPLE 8

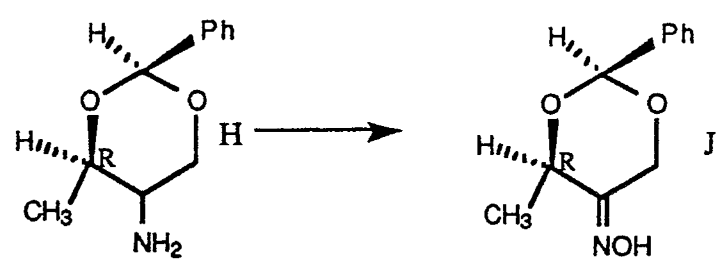

H → J

Add 3 g of the amine product of formula H from Example 7 above, 1 g of $Na_2WO_4$ and 50 ml of 25% aqueous $H_2O_2$ to 50 ml of methyl alcohol. Stir the reaction mixture for about 3 hrs. Partition the reaction mixture with $H_2O/CH_2Cl_2$, then dry the methylene chloride portion with $Na_2SO_4$. Evaporate the methylene chloride to give the desired product of formula J as a crystalline solid. Yield: 2.7 g (84%).

EXAMPLE 9

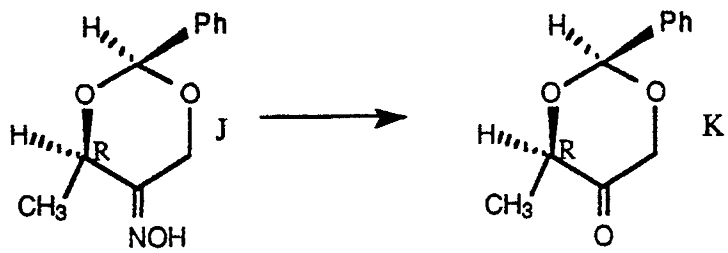

J K

Add 1 g of the oxime of formula J from Example 8 above, 1.5 g $NaNO_2$, 8.3 ml of acetic acid, 15 ml THF and 15 ml of water to a flask. Stir the reaction mixture for about 2 hrs. Partition the reaction mixture with $H_2O/CH_2Cl_2$. Dry the methylene chloride portion with $MgSO_4$. Evaporate the methylene chloride to give the product of formula K as a crystalline solid. Yield: 793 mg (85%).

EXAMPLE 10

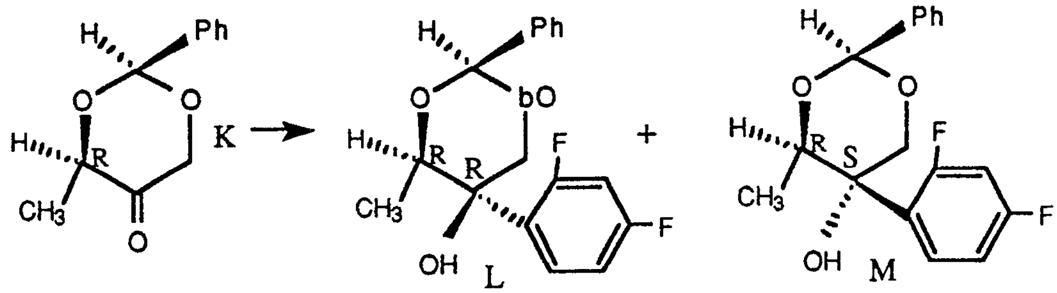

K L + M

Add magnesium (1 g) to a flask and heat with a $N_2$ purge. Cool the flask to room temperature and add 10 ml of THF and 8 g of 1-bromo-2,4-difluorobenzene. Once the reaction starts, add 40 ml of THF and control the exotherm with a water bath. Let the reaction mixture stir overnight to give the desired Grignard reagent.

Add the Grignard reagent (1.25 eq.in 2 ml THF solution) to a flask under nitrogen. Cool the flask to 0° C and add the product of formula K from Example 9 above (250 mg) in 5 ml of THF over about 1 minute. Stir the reaction mixture for about 1 half hr. at room temperature. Add 1 ml of aqueous saturated ammonium chloride solution and stir. Extract with $CH_2Cl_2$. Dry the extract with $Na_2SO_4$. Remove the solvent by vacuum distillation and purify the residue on a silica column using $CH_2Cl_2$/hexane (50/50) to provide 165 mg of the R,R isomer of formula L. The R,S isomer of formula M was also isolated in approximately 40 mg yield.

EXAMPLE 11

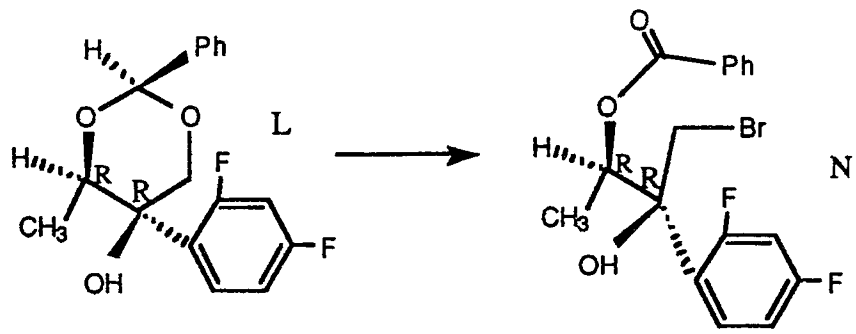

L N

Add 63 mg of the R,R compound of Formula L from Example above, 40 mg of N-bromo-succinamide (NBS), and 2 mg of benzoyl peroxide to 2 ml of carbon tetrachloride. Expose the reaction mixture to a UV lamp for one half hr. Purify the reaction mixture by silica gel chromatography using 10% ethylacetate/hexane to provide the bromohydrin product of formula N above. Yield: 27 mg.

EXAMPLE 12

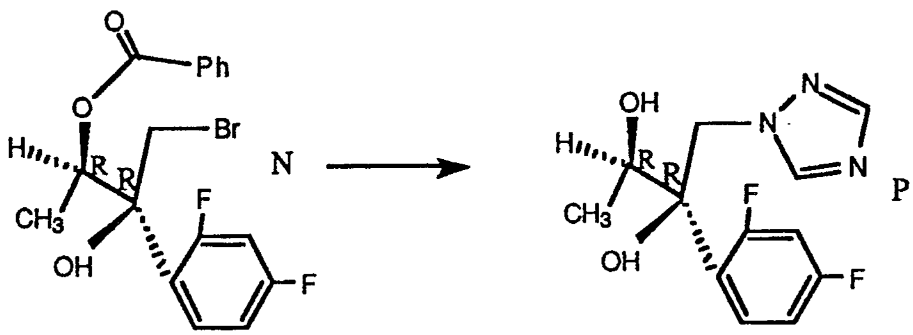

N P

Add sodium triazole (100 mg) to 1 ml of dimethylformamide containing 25 mg of the bromohydrin of formula N above. Heat the reaction mixture to 80 °C for about 2 hrs. and cool to room temperature. Partition the reaction mixture with $H_2O/CH_2Cl_2$. Remove the methylene chloride to give a residue. Add 5 ml of methyl alcohol and 3 drops of a 50% aqueous sodium hydroxide solution. After about 1 hour, remove the methyl alcohol with a $N_2$ stream. Purify the residue obtained by chromatography on a silica column using a gradient eluant of 100% $CH_2Cl_2$ → 5% $CH_3OH/CH_2Cl_2$ to yield 5 mg of the desired product of formula P above.

EXAMPLE 13

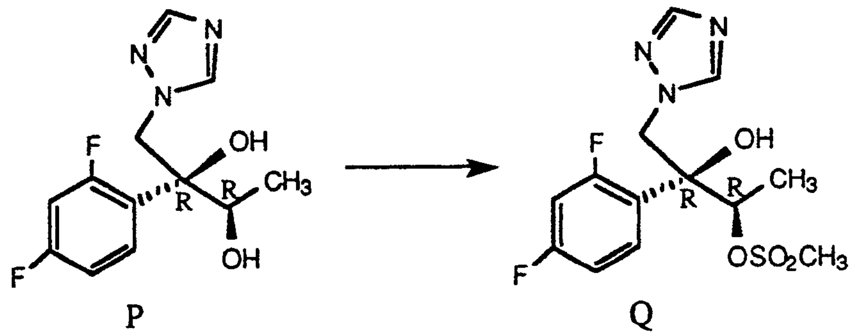

P Q

Add 259 mg of the compound of formula P above, 2.5 ml of dry methylene chloride, and 0.4 ml of triethylamine. Cool the resulting solution to 5° C and add dropwise 0.1 ml of mesylchloride. Remove the cooling bath and stir at room temperature until the reaction is complete as indicated by TLC. Dilute the reaction mixture with methylene chloride, wash twice with water, and dry over sodium sulfate. Remove the solvent by rotovap to yield 372 mg of the mesylated compound of formula Q above.

EXAMPLE 14

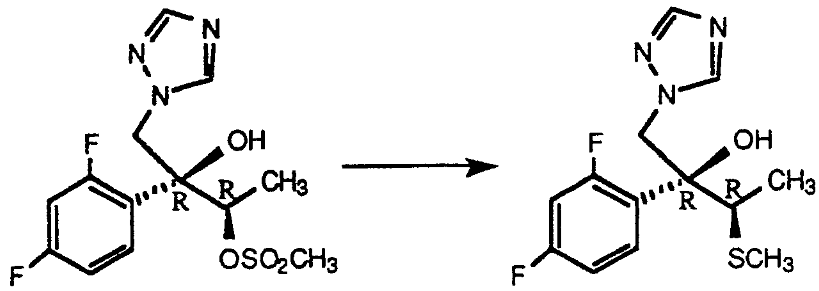

Q R

Add 372 mg of the mesylate of formula Q above to 1.3 ml dimethylsulfoxide. Add 1.4 ml of 15% aqueous sodium methyl sulfide at room temperature. The temperature rises to about 45° C. Warm the reaction solution to 55° C and stir at this temperature for 2 hours. The product precipitates out of solution. When the reaction is complete as indicated by TLC, add 3.3 ml of ice water. Let the reaction mixture stir at -5° C for 15 minutes. Filter off the product, wash with ice water, then once with hexane. Dry the product in a vacuum oven at 40° C. to yield 232 mg of the compound of formula R above.

EXAMPLE 15

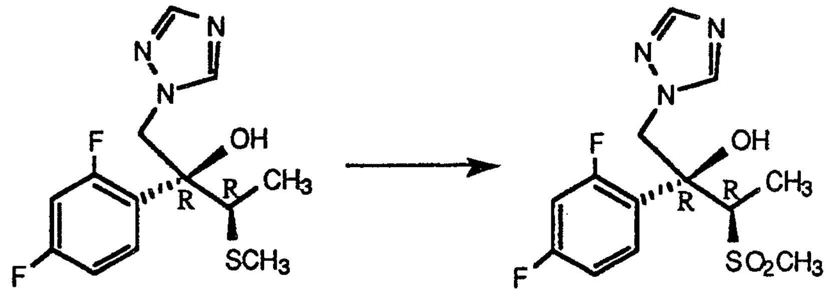

R S

Dissolve 220 mg of the compound of formula R above in 1.3 ml chloroform. Cool the solution to 5° C and add portionwise 299 mg of meta-chloro-perbenzoic acid (85%). Remove the cooling bath and let the reaction mixture stir at room temperature for 2 hours. When TLC indicates the reaction is complete, the insolubles are filtered off. Wash the precipitate once with methylene chloride. The filtrate is washed with 10% sodium carbonate solution and then with brine. Dry the solution over sodium sulfate, and evaporate to dryness on a rotovap. Triturate the residue with ethyl ether to produce a white crystalline solid which is filtered to yield 223 mg crude product. Crystallize the crude product in acetonitrile to yield117 mg of the compound of formula S above.

EXAMPLE 16

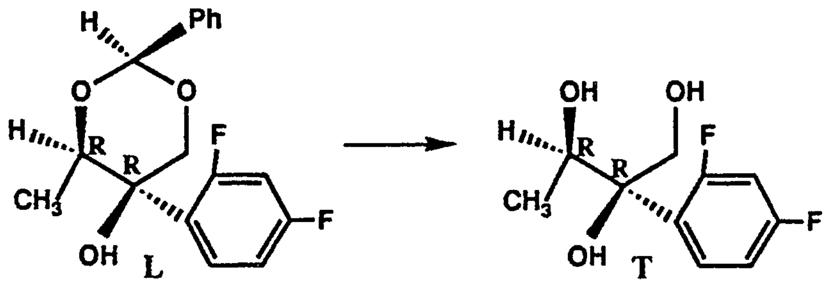

L T

Treat the RR isomer of formula L above with hydrogen and palladium-carbon in the presence of acetic acid at 25° C for 45 minutes to yield the triol of formula T above.

EXAMPLE 17

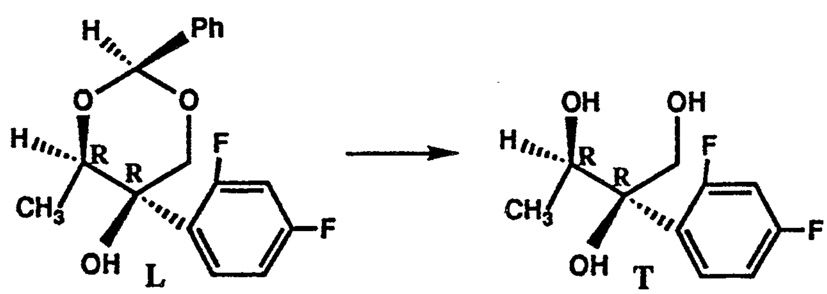

L T

Treat the RR isomer product of Example 10 above with aqueous $H_2SO_4$ (1N) in methanol at room temperature for 2 hrs. to yield the triol of formula T above.

EXAMPLE 18

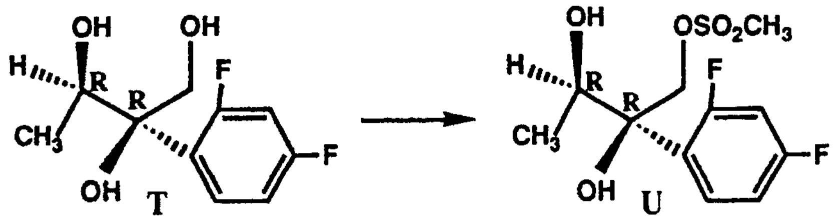

T U

Dissolve the triol from Example 16 above in methylene chloride. Add triethylamine (1.1 equivalents), cool to 0° C, and add 1 equivalent of methane sulfonyl chloride slowly. Stir the reaction mixture for one half hour, partition with water/methylene chloride and dry the methylene chloride fraction with $Na_2SO_4$. Evaporate the methylene chloride to give a crude product of formula U above.

EXAMPLE 19

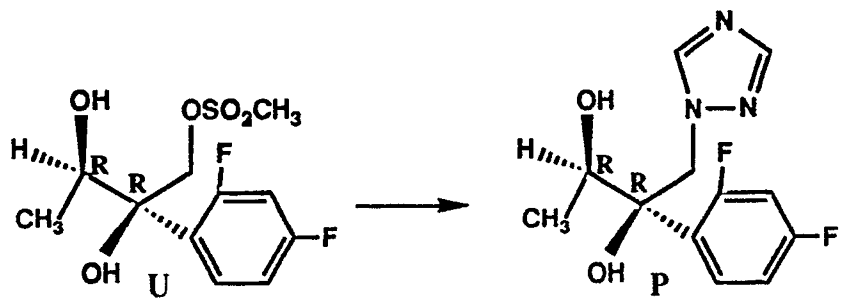

U P

Add the product of Example 18 above to 2 equivalents of sodium triazole in dimethylformamide. Heat the reaction mixture to 80° C for 2 hours, cool to room temperature and partition the reaction mixture with $H_2O/CH_2Cl_2$. Remove the methylene chloride to give a residue of formula W above and purify as set forth in Example 12 above.

Example 20

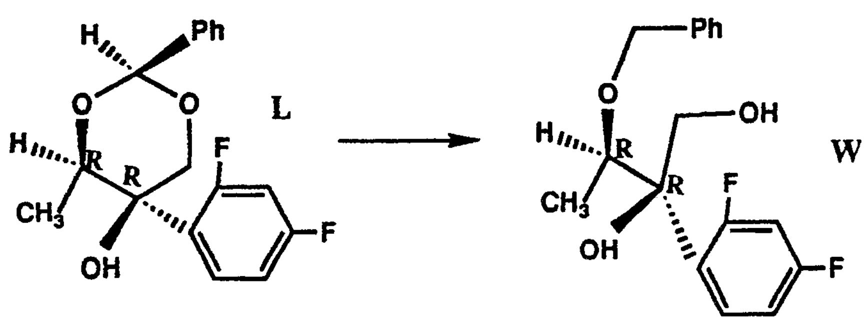

L W

Add the benzylidene acetal of formula L and $NaCNBH_3$ to $CH_3CN$ (4 ml). Add $(CH_3)_3SiCl$ to reaction

mixture as a neat liquid slowly (~3 min.). Stir the reaction for 3 days. Quench the reaction with 1 ml of $CH_3CO_2H$ and 1 ml of $H_2O$. Partition the reaction with $H_2O/CH_2Cl_2$. Dry the $CH_2Cl_2$ layer with $Na_2SO_4$ and purify the product of formula W with $CH_2Cl_2$ solution on a silica column using an elution gradient of 100% $CH_2Cl_2$ → 5% $CH_3OH/CH_2Cl_2$. Yield of product = 15 mg.

Example 21

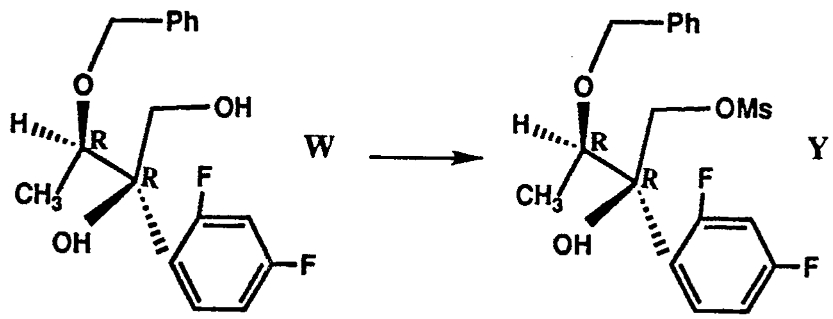

W

Y

React the above diol benzyl ether of formula W from EXAMPLE 20 above in methylene chloride (5 ml) with methane sulfonyl chloride (1.1 equivalent) and triethylamine (1.1 equivalent) at 10 °C for 1/2 hr. Partition the reaction mixture with water and methylene chloride. Extract and separate the methylene chloride layer from water to obtain the primary mesylate of the formula Y above.

While the present invention has been described in connection with the foregoing examples, many variations and alternatives are possible. All such variations and alternatives are intended to be within the scope of the following claims.

## Claims

1. A compound of the structural formula III or Va having an asymmetric center as indicated by an asterisk (*) and being substantially free of its opposite enantiomeric form:

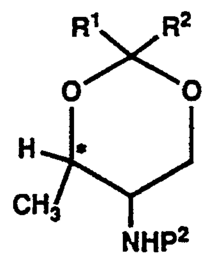

III or

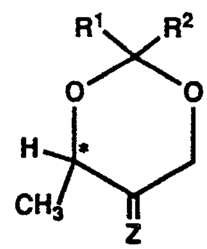

Va

wherein:

Z represents O or $NOP^1$;

$P^1$ is

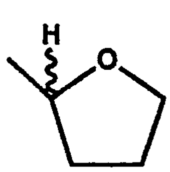,

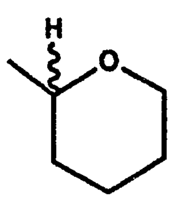,

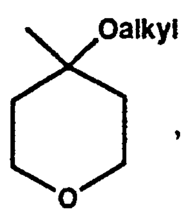,

H, alkyl, benzyl, substituted benzyl, alkoxy-substituted phenyl, alkyloxyalkyl, alkylthioalkyl or R′R″R‴silyl

where R′, R″ and R‴ may be the same or different and each is selected from alkyl, cycloalkyl or phenyl; $R^1$ and $R^2$ may be the same or different and each independently represents H, alkyl, phenyl, substituted phenyl or $R^1$ and $R^2$ together with the carbon to which they are attached represent the group

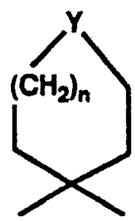

Y is (O, $S(O)_p$ or ($CR^aR^b$);
$R^a$ and $R^b$ may be the same or different and each is independently selected from H, alkyl, alkoxy, phenyl, substituted phenyl or -COOH or an alkyl, phenyl or substituted phenyl ester thereof,
p is 0, 1 or 2;
n is 0, 1 or 2; and
$P^2$ is H or an amino protecting group.

2. A compound according to claim 1, wherein $R^1$ is H, $R^2$ is phenyl or substituted phenyl, Z is O or NOH, and wherein $P^2$ is selected from H, benzyl, substituted benzyl, substituted phenyl, acyl, alkoxycarbonyl, halo-substituted alkoxycarbonyl, or unsubstituted or substituted allyloxycarbonyl.

3. A compound according to claim 1 or 2, wherein the asymmetric center indicated by the asterisk (*) is in the R absolute stereochemical configuration.

4. A compound of the structural formula VII

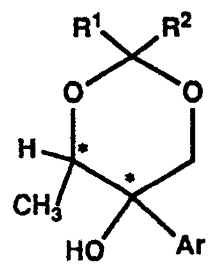

VII

wherein both of the asymmetric carbon centers indicated by the asterisk (*) have the same absolute stereochemical configuration, and wherein said compound is substantially free from compounds having other absolute stereochemical configurations at such carbon centers:
wherein:
Ar represents phenyl, substituted phenyl, 2- or 3-thienyl, substituted 2- or 3-thienyl, 2- or 3-furanyl, substituted 2- or 3-furanyl, 2-, 4-or 5-imidazolyl, 3-or 5-(1,2,4-triazolyl), 5-tetrazolyl, 2-, 4-or 5-thiazolyl, 2-, 4- or 5-oxazolyl, 3-, 4-or 5-isoxazolyl, 2-,3-or 4-pyridinyl, 2-or 3-pyrrolyl, 3-or 4-pyrrazolyl, 2-benzimidazolyl, 2-benzthiazolyl, or 2-, 4- or 6- purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl;
$R^1$ and $R^2$ may be the same or different and each independently represents H, alkyl, phenyl, substituted phenyl or $R^1$ and $R^2$ together with the carbon to which they are attached represent the group

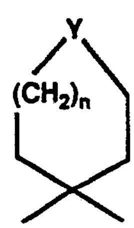

Y is (O, $S(O)_p$ or ($CR^aR^b$):

$R^a$ and $R^b$ may be the same or different and each is selected from H, alkyl, alkoxy, phenyl, substituted phenyl, -COOH or an alkyl, phenyl or substituted phenyl ester thereof;
p is 0, 1 or 2; and
n is 0, 1 or 2.

5. A compound according to claim 4, wherein $R^1$ is H, $R^2$ is phenyl or substituted phenyl, and Ar is phenyl or substituted phenyl.

6. A compound according to any one of the previous claims wherein Ar is 2,4-difluorophenyl.

7. A compound according to claim 4, wherein the asymmetric centers indicated by the asterisks (*) are both in their R absolute stereochemical configurations.

8. A process for preparing a compound of formula III set forth in claim 1 comprising reacting a compound of the formula I having an asymmetric center as indicated by the asterisk (*) and being substantially free of its opposite enantiomeric form:

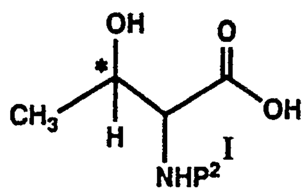

I

or an ester thereof with reducing agent to form a compound of the formula II.

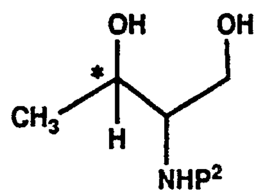

II;

and reacting the compound of formula II with an acetal-or ketal-forming agent of the formula $R^1R^2C=O$, $R^1R^2C(Oalkyl)_2$ or $R^2CHO$ to form a compound of the formula III

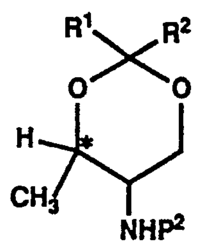

III,

wherein:
$R^1$ and $R^2$ may be the same or different and each independently represents H, alkyl, phenyl, substituted phenyl or $R^1$ and $R^2$ together with the carbon to which they are attached represent the group

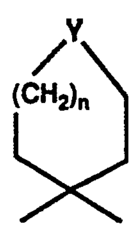

Y is (O, $S(O)_p$ or ($CR^aR^b$); $R^a$ and $R^b$ may be the same or different and each is selected from H, alkyl, alkoxy, phenyl, substituted phenyl, -COOH or an alkyl, phenyl or substituted phenyl ester thereof;

p is 0, 1 or 2;
n is 0, 1 or 2 and
$P^2$ represents H or an amino protecting group.
9. A process according to claim 8, further comprising
(a) reacting the compound of formula III with a deprotecting agent, when $P^2$ is not H, to form a compound of formula IV

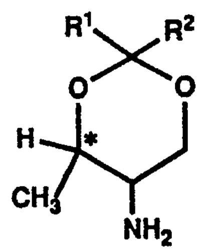 IV;

and (b) reacting the compound of formula IV with an oxidizing agent, followed, if desired, by reaction with a protective agent $P^1$ when $P^1$ is not H, to form a compound of the formula V

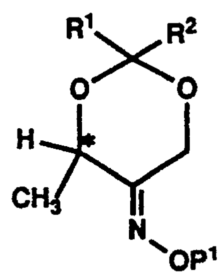 V,

wherein $P^1$ represents

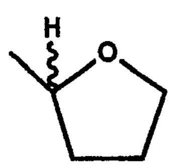 , 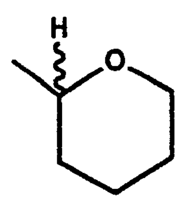 , 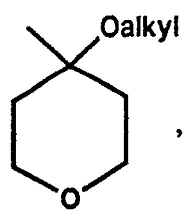 ,

H, alkyl, alkyloxyalkyl, alkylthioalkyl or $R'R''R'''$silyl where $R'$, $R'$ and $R'''$ may be the same or different and each is selected from alkyl, cycloalkyl or phenyl.
10. A process according to claim 9, further comprising reacting the compound of formula V with an oxidizing agent to form a compound of formula VI

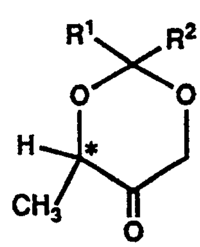 VI.

11. A process according to claim 10, further comprising reacting the compound of formula VI with a compound of the formula ArM wherein M represents a metallic group and Ar is a defined herein below to form a compound of formula VII and/or VIIa:

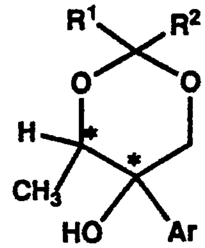 VII or 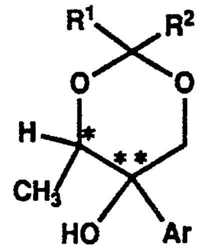 VIIa.

wherein:

the carbon centers having the single asterisk (*) and the double asterisks (**) indicate asymmetric carbon centers, with the double asterisked (**) carbon center having the opposite absolute stereo-chemical configuration as the single asterisked (*) carbon center; and

Ar represents phenyl, substituted phenyl, 2- or 3-thienyl, substituted 2- or 3-thienyl, 2- or 3-furanyl, substituted 2- or 3-furanyl, 2-, 4-or 5-imidazolyl, 3-or 5-(1,2,4-triazolyl), 5-tetrazolyl, 2-,4-or 5-thiazolyl, 2-, 4- or 5-oxazolyl, 3-, 4-or 5-isoxazolyl, 2-,3-or 4-pyridinyl, 2-or 3-pyrrolyl, 3-or 4-pyrrazolyl, 2-benzimidazolyl, 2-benzthiazolyl, or 2-, 4- or 6- purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl, and wherein $R^1$ and $R^2$ are as defined in claim 8.

12. A process according to claim 11, further comprising reacting the compound of formula VII (when $R^1$ is H) with an oxidizing agent (when $R^1$ is H) to form a compound of formula IX where $Z^1$ is O and X is halo, with a reducing agent to form a compound of formula IX where $Z^1$ is $H_2$ and X is OH, or with a hydrolyzing agent to form a triol of the formula IXa:

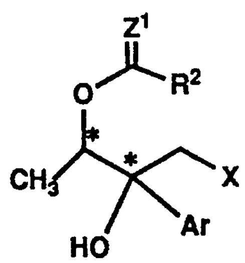 IX or 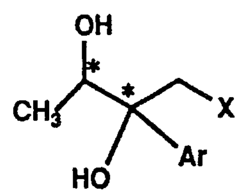 IXa;

converting any primary hydroxy group in formula IX or IXa to a leaving group; and reacting such leaving group with an $Ar^1$ anion to form a compound of the formula XII or XIII, respectively:

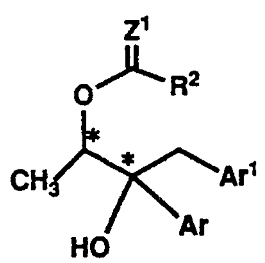 XII or 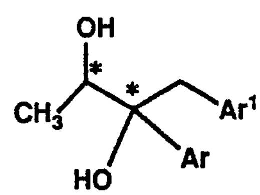 XIII,

wherein $Ar^1$ represents 1-(1,2,4-triazolyl), 1-(1,3,4-triazolyl) or 1-imidazolyl and wherein $Z^1$ represents $H_2$ or O.

13. A process according to claim 11, further comprising reacting the compound of formula VIIa with a mesylating agent to form a compound of the formula X wherein Ms represents a mesyl group:

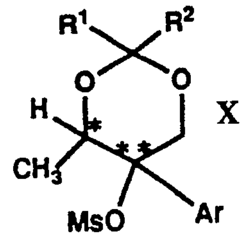

X;

reacting the compound of formula X (when $R^1$ is H) with an oxidizing agent followed by base treatment to form a compound of the formula XI where $Z^1$ is O, with a reducing agent (when $R^1$ is H) followed by base treatment to form a compound of formula XI where $Z^1$ is $H_2$, or with a hydrolyzing agent followed by base treatment to form a compound of formula XIa:

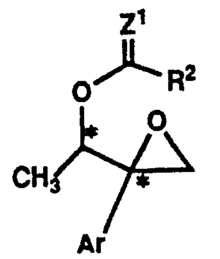

XI or

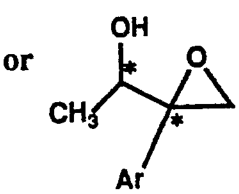

XIa,

wherein the absolute stereochemistry of the asymmetric center at the Ar carbon in formula XI or XIa is inverted with respect to the absolute configuration of the double asterisked carbon (**) in formula X; and reacting the compound of the formula XI with an $Ar^1$ anion to form a compound of the formula XII

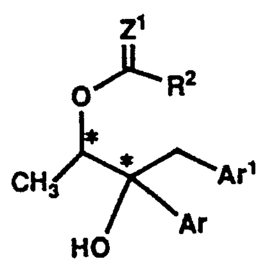

XII,

wherein:

$Ar^1$ represents 1-(1,2,4-triazolyl), 1-(1,3,4-triazolyl) or 1-imidazoyl; and

$Z^1$ represents $H_2$ or O.

14. A process according to claim 9, further comprising reacting the compound of formula V with a compound of the formula ArM wherein M is a metallic group to form a compound of formula VIII

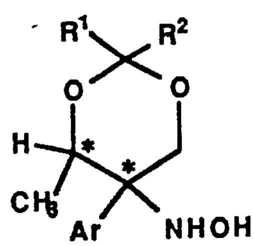

VIII,

wherein both the asterisked asymmetric centers have the same absolute stereochemical configuration; and reacting the compound of the formula VIII with a nitrosating agent to form a compound of the formula VII

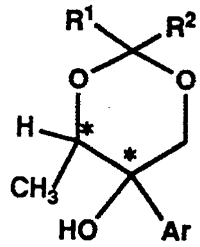

VII,

wherein:

Ar represents phenyl, substituted phenyl, 2- or 3-thienyl, substituted 2- or 3-thienyl, 2- or 3-furanyl, substituted 2- or 3-furanyl, 2-, 4-or 5-imidazolyl, 3-or 5-(1,2,4-triazolyl), 5-tetrazolyl, 2- ,4-or 5-thiazolyl, 2-, 4- or 5-oxazolyl, 3-, 4-or 5-isoxazolyl, 2-,3-or 4-pyridinyl, 2-or 3-pyrrolyl, 3-or 4-pyrrazolyl, 2-benzimidazolyl, 2-benzthiazolyl, or 2-, 4- or 6- purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl, and $R^1$ and $R^2$ are as defined in claim 8.

15. A process according to claim 12, further comprising reacting the compound of the formula XII with a hydroxy deprotecting agent to form a compound of the formula XIII.

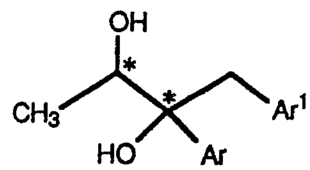

XIII

16. A process of preparing a compound of the formula XVI

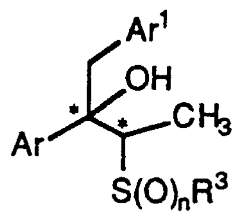

XVI;

wherein both asymmetric centers indicated by the asterisk (*) are substantially free of the opposite enantiomeric forms which comprises applying methods known per se to a compound of the formula XIII

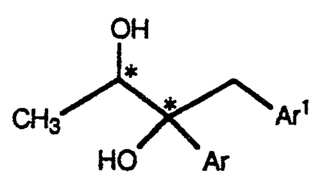

XIII

wherein both of the asymmetric carbon centers indicated by the asterisk (*), have the same absolute stereochemical configuration, and wherein said compound is substantially free from compounds having other absolute stereochemical configurations at such carbon centers, wherein Ar represents phenyl, substituted phenyl, 2- or 3-thienyl substituted 2- or 3-thienyl, 2- or 3-furanyl, substituted 2- or 3-furanyl, 2- ,4-or 5-imidazolyl, 3-or 5-(1,24-triazolyl), 5-tetrazolyl, 2-,4-or 5-thiazolyl, 2- ,4-or 5-oxazolyl, 3- ,4-or 5-isoxazolyl, 2-,3-or 4-pyridinyl, 2-or 3-pytrrolyl, 3-or 4-pyrrazolyl, 2-benzimidazolyl, 2-benzthiazolyl, or 2-,4- or

6-purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or subsituted phenyl; and
$Ar^1$ represents 1-(1,2,4-triazolyl), 1-(1,3,4-triazolyl) or 1-imidazolyl; $R^3$ is alkyl, cycloalkyl or cycloalkylalkyl; and wherein n is 1 or 2.

17. The process of claim 16 wherein the methods known per se comprise.

(a) reacting the compound of formula XIII with a mesylating agent to form a compound of the formula XIV

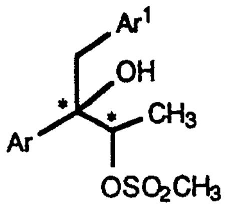

XIV;

(b) reacting the compound of the formula XIV with mercaptan anion of the formula $R^3S^-$ to form a compound of the formula XV

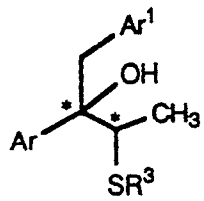

XV;

and reacting (c) the compound of the formula XV with an oxidizing agent to form a compound of the formula XVI

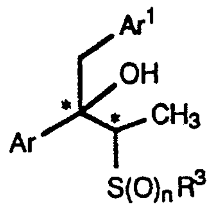

XVI;

wherein Ar, $Ar^1$ $R^3$ and n are as defined in claim 16

European Patent Office

# EUROPEAN SEARCH REPORT

Application Number

EP 90 30 9475

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SYNTHESIS, no. 7, 17th July 1989, pages 493-496, Georg Thieme Verlag, Stuttgart, DE; D. ENDERS et al.: "Enantioselective alkylation of 2,2-dimethyl-1,3-dioxan-5-one using the SAMP-/RAMP-hydrazone method" * The whole article, especially page 493, formula 5; page 495, table 3 * | 1 | C 07 D 319/06<br>C 07 D 407/12<br>C 07 D 249/08<br>C 07 C 69/78<br>C 07 C 33/46<br>C 07 C 43/178<br>C 07 C 309/66 |
| Y | EP-A-0 068 709 (STAUFFER CHEM.) * Claims * | 1 | |
| Y | EP-A-0 172 656 (ANGUS CHEM.) * Claims * | 1 | |
| Y | EP-A-0 322 800 (SUMITOMO) * The whole document * | 1,16,17 | |
| A | EP-A-0 244 143 (I.C.I.) * Claims * | 1 | |
| | | | **TECHNICAL FIELDS SEARCHED (Int. Cl.5)** |
| | | | C 07 D 319/00<br>C 07 D 407/00<br>C 07 D 249/00<br>C 07 C 69/00<br>C 07 C 33/00<br>C 07 C 43/00<br>C 07 C 309/00 |

The present search report has been drawn up for all claims

| Place of search | Date of completion of search | Examiner |
|---|---|---|
| The Hague | 26 November 90 | CHOULY J. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document